# EUROPEAN PATENT APPLICATION

(11) **EP 4 431 121 A1**
(43) Date of publication of application: **18.09.2024**
(21) Application number: 22906881.2
(22) Date of filing: 27.01.2022
(51) Int. Cl.: A61L 2/10, F24F 13/22

(54) **STERILIZATION SYSTEM, AIR CONDITIONER, STERILIZATION METHOD, AND STERILIZATION PROGRAM**

(30) Priority: 15.12.2021 JP 2021203343
(71) Applicant: MITSUBISHI HEAVY INDUSTRIES THERMAL SYSTEMS, LTD., Tokyo 100-8332 (JP)
(72) Inventor: MATSUMOTO, Soichiro, Tokyo 100-8332 (JP); TANAKA, Daisuke, Tokyo 100-8332 (JP); TAKEUCHI, Yasue, Tokyo 100-8332 (JP); OTA, Masahiro, Tokyo 100-8332 (JP)
(74) Representative: Cabinet Beau de Loménie
(86) International application number: PCT/JP2022/003131
(87) International publication number: WO 2023/112344

(57) **Abstract**

Provided are a sterilization system, an air conditioner, a sterilization method, and a sterilization program which make it possible to extend the life of a UV irradiation device and inhibit microorganisms in drain water. The sterilization system comprises an irradiation control unit that controls intermittent irradiation in which: irradiation is performed for an irradiation time that is not more than 22% of a reference time, said reference time being the time for which a prescribed sterilization rate is achieved when continuously irradiating drain water in a drain pan (3) of an air conditioner (1) with UV light from an LED (6); the irradiation is stopped for a prescribed pause time after the irradiation time; and irradiation is performed after the pause time.

## Description

### Technical Field

The present disclosure relates to a sterilization system, an air conditioner, a sterilization method, and a sterilization program.

### Background Art

In an air conditioner, an indoor unit is provided with a drain pan and drain water accumulated in the drain pan is discharged by a drain pump (for example, PTL 1).

### Citation List

### Patent Literature

[PTL 1] Japanese Unexamined Patent Application Publication No. 2008-190753

### Summary of Invention

### Technical Problem

In drain water remaining in a drain pan, proliferation of microorganisms (bacteria, mold, or the like) is likely to occur and metabolites of the microorganisms may become slime. In such a case, a flow path for the drain water may be blocked or a drain pump may be clogged.

A method of irradiating drain water with UV-C (LED) light to reduce the number of microorganisms has been examined. However, the life of an LED is short in many cases. For example, in a case where a cooling operation is performed for 24 hours and irradiation with UV-C light is performed constantly, the LED may reach the end of the life thereof in approximately one year. In a case where the LED reaches the end of the life thereof, a replacement operation needs to be performed.

The present disclosure has been made in view of such circumstances and an object of the present disclosure is to provide a sterilization system, an air conditioner, a sterilization method, and a sterilization program with which it is possible to suppress microorganisms in drain water while extending the life of a UV irradiation device. Solution to Problem

According to a first aspect of the present disclosure, there is provided a sterilization system including an irradiation control unit that controls intermittent irradiation in which irradiation is performed for an irradiation time of which a length is equal to or shorter than 22% of a length of a reference time, the irradiation is stopped for a predetermined stoppage time after the irradiation time, and the irradiation is performed after the stoppage time, the reference time being a time taken for a predetermined sterilization rate to be reached in a case where drain water in a drain pan of an air conditioner is continuously irradiated with UV light by a UV irradiation device.

According to a second aspect of the present disclosure, there is provided a sterilization method according to the present disclosure includes: performing irradiation for an irradiation time of which a length is equal to or shorter than 22% of a length of a reference time, the reference time being a time taken for a predetermined sterilization rate to be reached in a case where drain water in a drain pan of an air conditioner is continuously irradiated with UV light by a UV irradiation device; stopping the irradiation for a predetermined stoppage time after the irradiation time; and performing the irradiation after the stoppage time.

According to a third aspect of the present disclosure, there is provided a sterilization program according to the present disclosure causes a computer to execute a process of performing irradiation for an irradiation time of which a length is equal to or shorter than 22% of a length of a reference time, stopping the irradiation for a predetermined stoppage time after the irradiation time, and performing the irradiation after the stoppage time, the reference time being a time taken for a predetermined sterilization rate to be reached in a case where drain water in a drain pan of an air conditioner is continuously irradiated with UV light by a UV irradiation device.

### Advantageous Effects of Invention

According to the present disclosure, it is possible to suppress microorganisms in drain water while extending the life of a UV irradiation device.

### Brief Description of Drawings

Fig. 1 is a view showing a schematic configuration of an air conditioner provided with a sterilization system according to a first embodiment of the present disclosure.
Fig. 2 is a schematic configuration diagram showing an example of the hardware configuration of a control device according to the first embodiment of the present disclosure.
Fig. 3 is a functional block diagram showing the functions of the control device according to the first embodiment of the present disclosure.
Fig. 4 is a diagram showing a relationship between an irradiation time and the number of residual bacteria according to the first embodiment of the present disclosure.
Fig. 5 is a diagram showing a relationship between the irradiation time and the number of residual bacteria in a reference example.
Fig. 6 is a flowchart showing an example of the procedure for a sterilization process according to the first embodiment of the present disclosure.
Fig. 7 is a functional block diagram showing the functions of a control device according to a third embodiment of the present disclosure.

### Description of Embodiments

### (First Embodiment)

Hereinafter, a first embodiment of a sterilization system, an air conditioner, a sterilization method, and a sterilization program according to the present disclosure will be described with reference to the drawings.

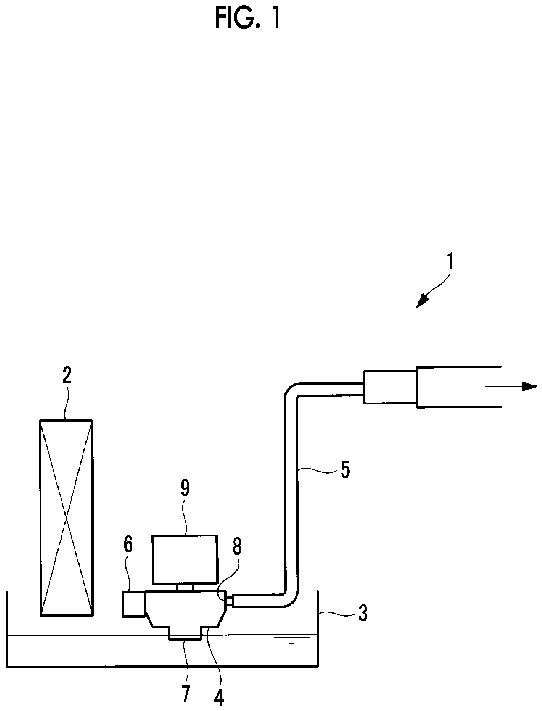

Fig. 1 is a view showing a schematic configuration of an air conditioner (air conditioner) 1 provided with a sterilization system according to a first embodiment of the present disclosure. As shown in Fig. 1, the air conditioner 1 according to the present embodiment includes a heat exchanger 2, a drain pan 3, a drain pump 4, a pipe 5, and a UV irradiation device (hereinafter, will be referred to as an "LED") 6 as main components. In addition, the air conditioner 1 is provided with a control device 10. In the present embodiment, the air conditioner 1 is, for example, a separated-type air conditioner including an indoor unit and an outdoor unit and the components shown in Fig. 1 are provided in the indoor unit which is a ceiling-embedded type indoor unit or the like. It will be assumed that the heat exchanger 2 is connected to a compressor (not shown), another heat exchanger (not shown), an expansion valve (not shown), and the like and forms a refrigerating cycle.

The heat exchanger 2 functions as an evaporator, and air is guided into the heat exchanger 2 by a blower (not shown). In addition, heat is exchanged between a refrigerant flowing in a heat transfer tube provided in the heat exchanger 2 and the air passing through the heat exchanger 2 (in the vicinity of the heat transfer tube), so that the air passing through the heat exchanger 2 is cooled. In the heat exchanger 2, liquefaction may occur due to condensation of moisture in the air since heat is absorbed from the air. Water (hereinafter, will be referred to as "drain water") generated due to such liquefaction drips, due to gravity, onto the drain pan 3 provided on a lower side.

The drain pan 3 stores the drain water generated in the heat exchanger 2 and is installed below the heat exchanger 2 so that the drain water dripping onto the drain pan 3 is stored. A water level gauge is provided in the drain pan 3. The water level gauge detects the storage water level of the drain water stored in the drain pan 3. The water level gauge prevents overflow. For example, cooling operation or dehumidification operation may be forcibly stopped based on a result obtained by using the water level gauge.

The drain pump 4 is, for example, a centrifugal pump, a rotary blade (not shown) is accommodated in a pump housing, and the drain pump 4 is provided with a suction port 7 that is formed at a lower portion of the pump housing and through which the drain water is sucked in and a discharge port 8 through which the drain water sucked in is discharged. Furthermore, a motor 9 that drives the rotary blade is connected to the drain pump 4. The drain pump 4 sucks up the drain water through the suction port 7 by using a centrifugal force that is generated as the motor 9 drives the rotary blade and the drain pump 4 discharges the drain water through the discharge port 8.

The pipe 5 is connected to the discharge port 8 of the drain pump 4. In addition, the drain water sucked up by the drain pump 4 is discharged to the outside of the indoor unit through the discharge port 8 and the pipe 5. The pipe 5 extends upward to guide the drain water to the outside of the indoor unit. That is, the drain pump 4 pumps up the drain water stored in the drain pan 3 to discharge the drain water to the outside of the indoor unit.

The UV irradiation device (LED) 6 is a device that performs irradiation with UV light (ultraviolet rays). Specifically, the LED 6 is a UV-C LED and performs irradiation with UV-C light (deep ultraviolet rays). The LED 6 is attached, for example, in the vicinity of the suction port of the drain pump 4.

The control device 10 controls the entire air conditioner 1. In addition, the control device 10 has a function (the sterilization system) of controlling the LED 6 to suppress microorganisms in the drain water.

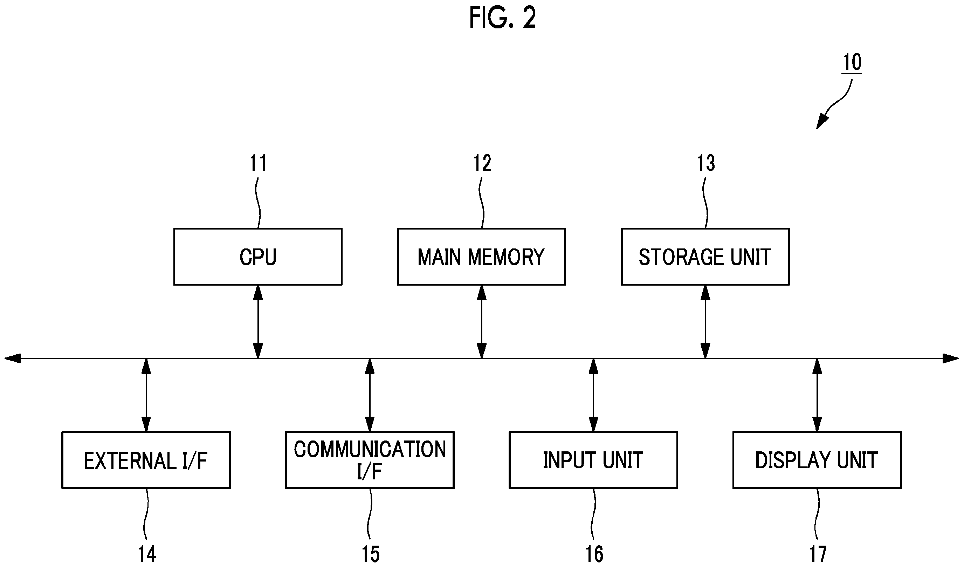

Fig. 2 is a schematic configuration diagram showing an example of the hardware configuration of the control device 10 according to an embodiment of the present disclosure. As shown in Fig. 1, the control device 10 is a so-called computer and includes a central processing unit (CPU) 11, a main memory 12, a storage unit 13, an external interface 14, a communication interface 15, an input unit 16, a display unit 17, and the like, for example. The above-described components are directly or indirectly connected to each other via a bus, and perform various processes in cooperation with each other.

For example, the CPU 11 controls the entire control device 10 by using an operating system (OS) stored in the storage unit 13 connected via the bus and performs various processes by executing various programs stored in the storage unit 13.

For example, the main memory 12 is composed of a writable memory such as a cache memory or a random-access memory (RAM), and is used as a work region for the reading of an execution program of the CPU 11, the writing of processing data of the execution program, or the like.

The storage unit 13 is a non-transitory storage medium (a non-transitory computer-readable storage medium), and is, for example, a read-only memory (ROM), a hard disk drive (HDD), a flash memory, or the like. The storage unit 13 stores, for example, an OS for controlling the entire device such as Windows (registered trademark), iOS (registered trademark), and Android (registered trademark), a basic input/output system (BIOS), various device drivers for hardware-operating peripheral equipment, various kinds of application software, various data, files, and the like. The storage unit 13 stores a program for realizing various processes and various data required for realizing various processes.

The external interface 14 is an interface for connection to an external device. Examples of the external device include an external monitor, a USB memory, and an external HDD. In an example shown in Fig. 1, only one external interface is shown. However, a plurality of external interfaces may be provided.

The communication interface 15 is connected to a network and communicates with other devices to function as an interface for transmission and reception of information.

For example, the communication interface 15 communicates with other devices in a wired or wireless manner. Examples of wireless communication include Bluetooth (registered trademark), Wi-Fi, and communication in which a dedicated communication protocol is used. Examples of wired communication include a wired local area network (LAN).

The input unit 16 is, for example, a user interface for issuing an instruction such as a keyboard, a mouse, and a touch pad.

The display unit 17 is, for example, a liquid-crystal display, an organic electroluminescence (EL) display, or the like. The display unit 17 may be a touch panel display overlaid with a touch panel.

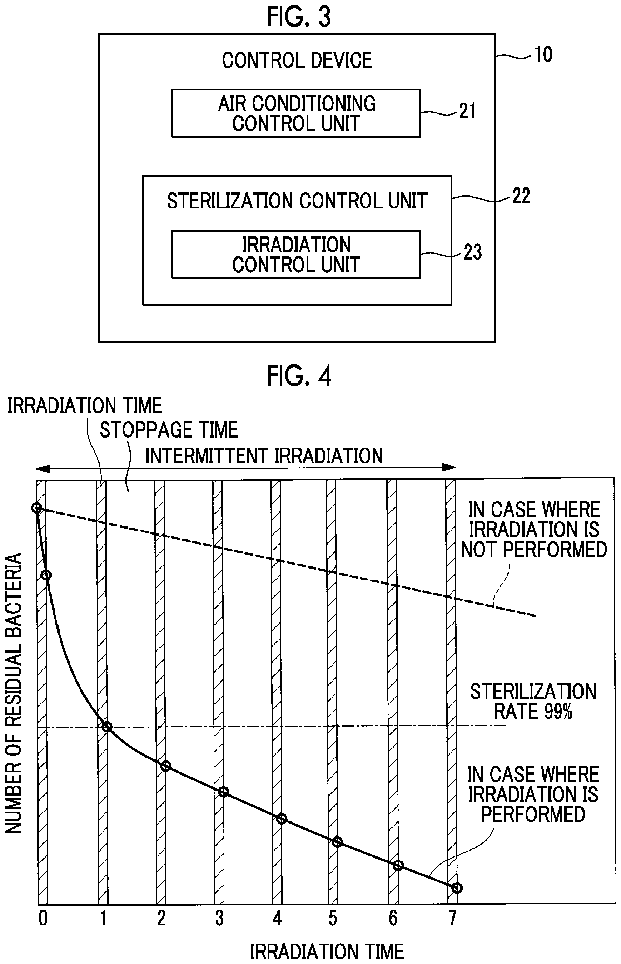

Fig. 3 is a functional block diagram showing the functions of the control device 10. As shown in Fig. 3, the control device 10 includes an air conditioning control unit 21 and a sterilization control unit (the sterilization system) 22. The sterilization control unit 22 includes an irradiation control unit 23. In the present embodiment, the control device 10 is configured to include the sterilization control unit 22. However, the sterilization control unit 22 may be provided separately from the control device 10. For example, the sterilization control unit 22 and the LED 6 may be configured as one module.

For example, the functions realized by the above-described units are realized by a processing circuitry. For example, a series of processes for realizing functions described below is stored in the storage unit 13 in the form of a program (for example, a route instruction data creating program), as an example. The CPU 11 reads the program into the main memory 12 and executes information processing and a calculation process to realize various functions.

The program may be installed in the storage unit 13 in advance, may be provided in a state of being stored in another computer-readable storage medium, or may be distributed via a wired or wireless communication unit. Examples of the computer-readable storage medium include a magnetic disk, a magneto-optical disk, a CD-ROM, a DVD-ROM, and a semiconductor memory.

The air conditioning control unit 21 performs various controls with respect to the air conditioner 1. Specifically, the air conditioning control unit 21 controls the refrigerating cycle by switching between operation modes such as heating operation, cooling operation, and dehumidification operation.

The irradiation control unit 23 controls irradiation performed by the LED 6 to suppress microorganisms in the drain water. Specifically, the irradiation control unit 23 performs irradiation for an irradiation time of which the length is equal to or shorter than 22% of the length of a reference time, the reference time being a time taken for a predetermined sterilization rate (disinfection rate) to be reached in a case where the drain water is continuously irradiated with UV light. Then, after the irradiation time elapses, the irradiation is stopped for a predetermined stoppage time and the irradiation is performed after the stoppage time elapses. That is, the irradiation control unit 23 controls intermittent irradiation performed with UV light. The predetermined sterilization rate is, for example, 99%, but can be optionally set.

Since drain water is likely to be generated in the case of the cooling operation or the dehumidification operation, an intermittent irradiation process is started during the cooling operation or the dehumidification operation or after stoppage of such operation.

Fig. 4 is a diagram showing a relationship between the irradiation time and the number of residual bacteria in the present embodiment. Fig. 4 shows an example of intermittent irradiation, and a specific process such as the irradiation time is not limited to that shown in Fig. 4. As shown in Fig. 4, the irradiation control unit 23 performs intermittent irradiation with UV light. Particularly, the irradiation control unit 23 repeats performing irradiation for the irradiation time and stopping the irradiation for the stoppage time. The number of times of repetition during the intermittent irradiation is not limited. Irradiation times of which the lengths are the same as each other and stoppage times of which the lengths are the same as each other are repeated. However, irradiation times of which the lengths are different from each other and stoppage times of which the lengths are different from each other may be repeated. Fig. 4 shows a change in number of residual bacteria in a case where irradiation is performed and a change in number of residual bacteria in a case where irradiation is not performed. The number of residual bacteria in the case where the irradiation is not performed is the number of residual bacteria in the case of natural extinction of microorganisms or the like without an increase in number thereof.

In Fig. 4, a case where each irradiation time is 10 minutes and each stoppage time is 50 minutes is shown as an example. In this way, the irradiation control unit 23 repeats performing irradiation for 10 minutes, stopping irradiation for 50 minutes thereafter, performing irradiation for 10 minutes thereafter, ... and so forth. The total number of times of irradiation is seven, and the total irradiation time is 70 minutes.

In a case where the intermittent irradiation is performed, as shown in Fig. 4, the number of residual bacteria is significantly reduced at the time of the first irradiation and the sterilization rate reaches 99% at the time of the second irradiation which is performed after stoppage (when 70 minutes elapse after the start). Thereafter, performing irradiation for a short time is intermittently repeated and thus the number of residual bacteria is effectively reduced.

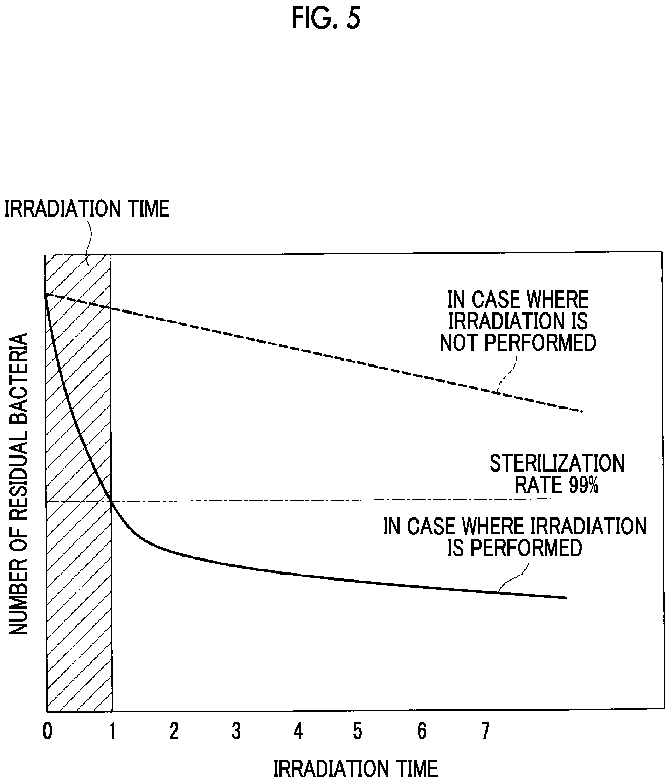

Fig. 5 is a diagram showing a relationship between the irradiation time and the number of residual bacteria in a reference example. In the reference example, irradiation with UV light is continuously performed for 70 minutes. Fig. 5 shows a change in number of residual bacteria in a case where irradiation is performed and a change in number of residual bacteria in a case where irradiation is not performed. Even in a case where irradiation is continuously performed for 70 minutes as shown in Fig. 5, the sterilization rate can reach 99%. However, in the present embodiment, as shown in Fig. 4, it is possible to make the sterilization rate reach 99% by performing irradiation for 10 minutes twice with a stoppage period (50 minutes) provided as an interval. That is, in a case where a sterilization rate of 99% is used as a reference, irradiation needs to be performed for 70 minutes in the case of continuous irradiation. However, it is sufficient to perform irradiation for a total of 20 minutes in the case of intermittent irradiation. In the case of intermittent irradiation as shown in Fig. 4, further sterilization can be performed in a case where irradiation is performed for a total of 70 minutes. Particularly, in a case where the LED 6 continuously performs irradiation for a long period of time, the output of the LED 6 may decrease due to an increase in temperature thereof. However, in the case of intermittent irradiation, the increase in temperature is suppressed because of the stoppage time and thus a time during which the output is high can be effectively used.

In a case where the intermittent irradiation is performed as described above, it is possible to suppress a UV irradiation time while effectively performing sterilization. Since the life of the LED 6 is determined by the total irradiation time, the life of the LED 6 can be extended in the case of intermittent operation.

Since there is a stoppage time, intermittent irradiation can result in effective sterilization in comparison with continuous irradiation because of natural convection of the drain water during the stoppage time.

Next, the setting of the irradiation time will be described.

The irradiation time is set based on the reference time. The reference time is a time taken for the predetermined sterilization rate to be reached in a case where irradiation with UV light is continuously performed. In the reference example shown in Fig. 5, the sterilization rate reaches 99% when irradiation with UV light is continuously performed for 70 minutes. That is, for a sterilization rate of 99%, the reference time is 70 minutes. The sterilization rate relating to a case where the reference time is set can be applied without being limited to 99%.

Here, as shown in Fig. 4, a sterilization effect can be achieved without continuous irradiation. Therefore, the irradiation time is set to be equal to or shorter than 22% of the reference time. Specifically, in a case where the reference time is 70 minutes, it is preferable that the irradiation time is 5 minutes or more and 15 minutes or less (10 minutes in the example shown in Fig. 4).

A time longer than the irradiation time is set as the stoppage time in the case of the intermittent operation. Specifically, the stoppage time is set to be equal to or longer than three times the irradiation time. In the example shown in Fig. 4, the stoppage time is 50 minutes. Even in a case where the stoppage time is longer than the irradiation time as described above, a sufficient sterilization effect can be achieved.

Next, the useful life of the LED 6 will be described.

The useful life is, for example, a rated life. The rated life may be an irradiation time taken for the output of the LED to reach 70% of the initial output thereof or an irradiation time taken for the output of the LED to reach 50% of the initial output thereof. That is, the rated life is set as a reference for replacement of the LED 6.

The useful life of the LED 6 is 5 or more and 10 or less in a case where the useful life of the air conditioner 1 is 10. The useful life of the air conditioner 1 is the useful life of the air conditioner 1 in a configuration other than the LED 6 (in a case where the LED 6 is not provided).

For example, the lower limit of the useful life of the LED 6 is set on the assumption that replacement is performed once until the end of the useful life of the air conditioner 1 is reached. For example, the upper limit of the useful life of the LED 6 is set on the assumption that replacement is not performed even once until the end of the useful life of the air conditioner 1 is reached.

Accordingly, it is possible to suppress replacement of the LED 6 being frequently performed until the end of the useful life of the air conditioner 1 is reached. For example, the LED 6 can be replaced once (or zero times) until the end of the useful life of the air conditioner 1 is reached.

Next, the amount of light of the LED 6 in a predetermined time will be described.

In the case of intermittent irradiation, the irradiation control unit 23 performs irradiation with a predetermined amount of light, which is equal to or greater than 5000 mW.sec and equal to or smaller than 90000 mW.sec, per unit time. The amount of light may be the amount of light from one LED or the total amount of light from a plurality of LEDs, and the configuration is not limited as long as irradiation with the predetermined amount of light is performed. For example, in a case where irradiation is performed for 10 minutes at 10 mW (min) and then irradiation is stopped for 60 minutes, 5143 mW.sec per unit time (60 minutes) is achieved. For example, in a case where irradiation is performed for 15 minutes at 50 mW (min) and then irradiation is stopped for 45 minutes, 90000 mW.sec per unit time (60 minutes) is achieved.

Since irradiation with the predetermined amount of light, which is equal to or greater than 5000 mW.sec and equal to or smaller than 90000 mW.sec, is performed per unit time in the case of intermittent irradiation, irradiation with an amount of light sufficient for sterilization can be performed.

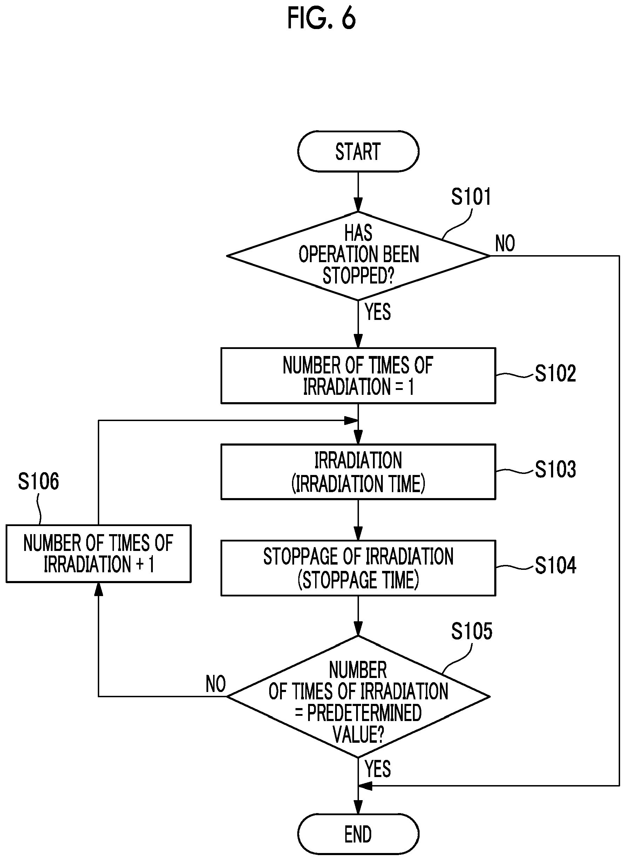

Next, an example of a sterilization process performed by the above-described control device 10 will be described with reference to Fig. 6. Fig. 6 is a flowchart showing an example of the procedure for a sterilization process according to the present embodiment. The flow shown in Fig. 6 is repeatedly executed, for example, at a predetermined control cycle. In an example shown in Fig. 6, a case where irradiation is performed 7 times in the form of intermittent irradiation will be described as an example.

First, it is determined whether or not the cooling operation or the dehumidification operation has been stopped (S101). Regarding S101, it may be determined whether or not a predetermined timing (for example, an operation continuation time) has been reached during the cooling operation or the dehumidification operation. That is, in S101, the result of the determination may be YES in a case where the cooling operation or the dehumidification operation is stopped (intermittent irradiation after stoppage of operation) or the result of the determination may be YES in a case where the predetermined timing is reached during the operation (intermittent irradiation during operation).

In a case where the result of the determination in S101 is NO, the process is terminated. In a case where the cooling operation or the dehumidification operation is stopped (the result of the determination in S101 is YES), an intermittent irradiation process is started. First, the number of times of irradiation is set to 1 (S102). Then, irradiation is performed for the irradiation time (S103) .

Next, irradiation is stopped for the stoppage time (S104). Next, it is determined whether or not the number of times of irradiation has reached a predetermined value (the number of times of irradiation = 7) (S105) . In a case where the result of the determination in S105 is NO, 1 is added to the number of times of irradiation (S106) and S103 is executed. In a case where the result of the determination in S105 is YES, it is determined that irradiation has been performed seven times and the process is terminated.

In the flow of Fig. 6, a case where irradiation is performed 7 times has been described. However, the number of times of irradiation is not limited. In the flow of Fig. 6, whether to terminate the process is performed based on the number of times of irradiation. However, the process may be terminated in a case where the total intermittent irradiation time reaches a predetermined time and a termination method is not limited.

As described above, in the case of a sterilization system, an air conditioner, a sterilization method, and a sterilization program according to the present embodiment, irradiation is performed for an irradiation time of which the length is equal to or shorter than 22% of the length of a reference time, the reference time being a time taken for a predetermined sterilization rate to be reached in a case where drain water is continuously irradiated with UV light. Then, the irradiation is stopped for a stoppage time and the irradiation is performed again thereafter. Accordingly, sterilization can be performed effectively even in a case where irradiation with UV light is not continuously performed for the reference time. In addition, since a time for which irradiation with UV light is performed is shortened, the life of the UV irradiation device can be extended.

### (Second Embodiment)

Next, a sterilization system, an air conditioner, a sterilization method, and a sterilization program according to a second embodiment of the present disclosure will be described.

In the present embodiment, a case where an intermittent operation process is executed again will be described. Hereinafter, the sterilization system, the air conditioner, the sterilization method, and the sterilization program according to the present embodiment will be described focusing on differences between the first embodiment and the present embodiment.

In the case of a long vacation or the like, the air conditioner 1 is not operated for a long period of time and microorganisms are likely to proliferate in a high-temperature and high-humidity environment. Therefore, the irradiation control unit 23 in the present embodiment performs intermittent irradiation in a case where a predetermined standby time elapses after the end of intermittent irradiation. That is, the irradiation control unit 23 performs the intermittent irradiation as shown in Fig. 4 again after the standby time elapses.

Particularly, in a case where the cooling operation or the dehumidification operation in the air conditioner 1 is not performed, microorganisms are likely to proliferate in the drain pan 3. Therefore, the irradiation control unit 23 performs intermittent irradiation in a case where the cooling operation or the dehumidification operation in the air conditioner 1 is not performed for the standby time after the end of intermittent irradiation. The standby time is set to be longer than a time for which an intermittent irradiation process is performed. For example, the standby time is set to 24 hours. In addition, a process of repeating the intermittent irradiation and the standby time is performed for a predetermined period of time. The predetermined period of time is set to, for example, 7 days in which drain water is expected to evaporate.

As described above, in the case of the sterilization system, the air conditioner, the sterilization method, and the sterilization program according to the present embodiment, since intermittent irradiation is performed when the predetermined standby time elapses after the end of intermittent irradiation, it is possible to suppress proliferation of microorganisms even in a case where there is a possibility that microorganisms proliferate after the end of intermittent irradiation. Although microorganisms are likely to proliferate in a case where the cooling operation or the dehumidification operation in the air conditioner 1 is not performed, since intermittent irradiation is performed in a case where the standby time elapses, it is possible to suppress proliferation of microorganisms.

### (Third Embodiment)

Next, a sterilization system, an air conditioner, a sterilization method, and a sterilization program according to a third embodiment of the present disclosure will be described.

In the present embodiment, a case where the drain pump 4 is driven will be described. Hereinafter, the sterilization system, the air conditioner, the sterilization method, and the sterilization program according to the present embodiment will be described focusing on differences between the first embodiment, the second embodiment, and the present embodiment.

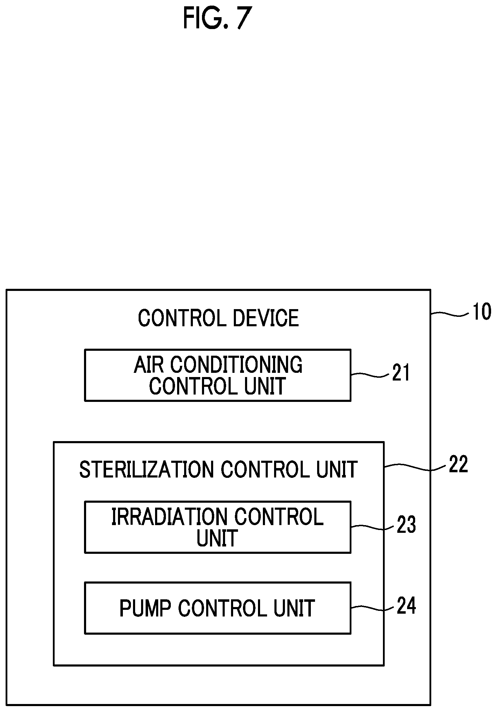

As shown in Fig. 7, the sterilization control unit 22 in the present embodiment includes a pump control unit 24. The pump control unit 24 operates, for a certain time, the drain pump 4 that sucks drain water in the drain pan 3.

For example, after stoppage of the cooling operation or the dehumidification operation in the air conditioner 1, the pump control unit 24 operates the drain pump 4 for a predetermined time (about 1 minute) before irradiation with UV light.

The drain water is sucked into the drain pump 4 as the drain pump 4 is driven. Then, in a case where the driving of the drain pump 4 is stopped, drain water that is sucked into the drain pump 4 and that is not discharged to the outside flows back to the drain pan 3. Accordingly, the drain water can be stirred.

As described above, in the case of the sterilization system, the air conditioner, the sterilization method, and the sterilization program according to the present embodiment, since the drain pump 4 that sucks drain water in the drain pan 3 is operated for a certain time, the drain water that is sucked into the drain pump 4 from the drain pan 3 and that is not completely sucked out flows back to the drain pan 3 and thus the drain water can be stirred. Accordingly, sterilization effect achieved by UV light can be improved.

### (Fourth Embodiment)

Next, a sterilization system, an air conditioner, a sterilization method, and a sterilization program according to a fourth embodiment of the present disclosure will be described.

In the present embodiment, a case where whether or not drain water is present is determined will be described. Hereinafter, the sterilization system, the air conditioner, the sterilization method, and the sterilization program according to the present embodiment will be described focusing on differences between the first embodiment, the second embodiment, the third embodiment, and the present embodiment.

In the present embodiment, the air conditioner 1 is provided with a sensor that detects the presence or absence of drain water in the drain pan 3. The sensor is, for example, an electrostatic sensor, a float switch, or the like. That is, the sensor detects that drain water is present in the drain pan 3.

In addition, the irradiation control unit 23 performs irradiation in a case where the sensor detects that drain water is present in the drain pan 3. That is, intermittent irradiation is performed after the sensor confirms that there is drain water.

The sensor may be provided as an individual device, or may be integrally configured with a module of the LED 6. For example, the sensor may be incorporated into a module into which the sterilization control unit 22 and the LED 6 are incorporated. In this case, the function of the sterilization system can be added by adding the module to the existing air conditioner 1.

As described above, in the case of the sterilization system, the air conditioner, the sterilization method, and the sterilization program according to the present embodiment, irradiation is performed in a case where the sensor detects that drain water is present in the drain pan 3. Therefore, unnecessary irradiation with UV light can be suppressed.

The present disclosure is not limited to only the embodiments described above, and various modifications can be made without departing from the scope of the invention. It is also possible to combine the embodiments with each other. In other words, the above-described first embodiment, the second embodiment, the third embodiment, and the fourth embodiment can be combined with each other.

The sterilization system, the air conditioner, the sterilization method, and the sterilization program described in the above-described embodiments are understood as follows, for example.

A sterilization system (22) according to the present disclosure includes an irradiation control unit (23) that controls intermittent irradiation in which irradiation is performed for an irradiation time of which a length is equal to or shorter than 22% of a length of a reference time, the irradiation is stopped for a predetermined stoppage time after the irradiation time, and the irradiation is performed after the stoppage time, the reference time being a time taken for a predetermined sterilization rate to be reached in a case where drain water in a drain pan (3) of an air conditioner (1) is continuously irradiated with UV light by a UV irradiation device (6).

In the case of the sterilization system according to the present disclosure, irradiation is performed for the irradiation time of which the length is equal to or shorter than 22% of the length of the reference time, the reference time being a time taken for the predetermined sterilization rate to be reached in a case where the drain water is continuously irradiated with UV light. Then, the irradiation is stopped for a stoppage time and the irradiation is performed again thereafter. Accordingly, sterilization can be performed effectively even in a case where irradiation with UV light is not continuously performed for the reference time. In addition, since a time for which irradiation with UV light is performed is shortened, the life of the UV irradiation device can be extended. The predetermined sterilization rate is, for example, 99%.

In the sterilization system according to the present disclosure, the irradiation control unit may repeat performing irradiation for the irradiation time and stopping irradiation for the stoppage time.

In the case of the sterilization system according to the present disclosure, since performing irradiation for the irradiation time and stopping irradiation for the stoppage time are repeated, it is possible to reduce a load on the UV irradiation device while suppressing a decrease in sterilization effect in comparison with a case where irradiation with UV light is continuously performed.

In the sterilization system according to the present disclosure, the stoppage time may be set to be equal to or longer than three times the irradiation time.

In the case of the sterilization system according to the present disclosure, even in a case where irradiation is stopped for the stoppage time equal to or longer than three times the irradiation time, it is possible to reduce a load on the UV irradiation device while suppressing a decrease in sterilization effect in comparison with a case where irradiation with UV light is continuously performed.

In the sterilization system according to the present disclosure, the irradiation control unit may perform irradiation with a predetermined amount of light, which is equal to or greater than 5000 mW.sec and equal to or smaller than 90000 mW.sec, per unit time in the case of the intermittent irradiation.

In the case of the sterilization system according to the present disclosure, since intermittent irradiation is performed with a predetermined amount of light, which is equal to or greater than 5000 mW.sec and equal to or smaller than 90000 mW.sec, per unit time in the case of the intermittent irradiation, it is possible to perform sterilization while suppressing the total amount of light in the case of the intermittent irradiation.

In the sterilization system according to the present disclosure, the irradiation control unit may perform the intermittent irradiation in a case where a predetermined standby time elapses after the end of the intermittent irradiation.

In the case of the sterilization system according to the present disclosure, since intermittent irradiation is performed when the predetermined standby time elapses after the end of intermittent irradiation, it is possible to suppress proliferation of microorganisms even in a case where there is a possibility that microorganisms proliferate after the end of intermittent irradiation.

In the sterilization system according to the present disclosure, the irradiation control unit may perform the intermittent irradiation in a case where cooling operation or dehumidification operation in the air conditioner is not performed for the standby time after the end of the intermittent irradiation.

In the case of the sterilization system according to the present disclosure, although microorganisms are likely to proliferate in a case where the cooling operation or the dehumidification operation in the air conditioner is not performed, since intermittent irradiation is performed in a case where the standby time elapses, it is possible to suppress proliferation of microorganisms.

The sterilization system according to the present disclosure may further include a pump control unit (24) that operates, for a certain time, a drain pump (4) that sucks the drain water in the drain pan.

In the case of the sterilization system according to the present disclosure, since the drain pump that sucks drain water in the drain pan is operated for the certain time, the drain water that is sucked into the drain pump from the drain pan and that is not completely sucked out flows back to the drain pan and thus the drain water can be stirred. Accordingly, sterilization effect achieved by UV light can be improved.

In the sterilization system according to the present disclosure, the irradiation control unit may perform irradiation in a case where a sensor detects that the drain water is present in the drain pan.

In the case of the sterilization system according to the present disclosure, irradiation is performed in a case where the sensor detects that drain water is present in the drain pan. Therefore, unnecessary irradiation with UV light can be suppressed.

An air conditioner according to the present disclosure includes a drain pan, a UV irradiation device, and the above-described sterilization system.

In the air conditioner according to the present disclosure, the useful life of the UV irradiation device may be 5 or more and 10 or less in a case where the useful life of the air conditioner is 10.

In the case of the air conditioner according to the present disclosure, it is possible to suppress replacement of the UV irradiation device being frequently performed until the end of the useful life of the air conditioner is reached. For example, the UV irradiation device can be replaced once (or zero times) until the end of the useful life of the air conditioner is reached.

In the air conditioner according to the present disclosure, the useful life may be a rated life.

In the case of the air conditioner according to the present disclosure, since the useful life is the rated life, a relationship between the useful life of the UV irradiation device and the useful life of the air conditioner can be appropriately set.

A sterilization method according to the present disclosure includes: performing irradiation for an irradiation time of which a length is equal to or shorter than 22% of a length of a reference time, the reference time being a time taken for a predetermined sterilization rate to be reached in a case where drain water in a drain pan of an air conditioner is continuously irradiated with UV light by a UV irradiation device; stopping the irradiation for a predetermined stoppage time after the irradiation time; and performing the irradiation after the stoppage time.

A sterilization program according to the present disclosure causes a computer to execute a process of performing irradiation for an irradiation time of which a length is equal to or shorter than 22% of a length of a reference time, stopping the irradiation for a predetermined stoppage time after the irradiation time, and performing the irradiation after the stoppage time, the reference time being a time taken for a predetermined sterilization rate to be reached in a case where drain water in a drain pan of an air conditioner is continuously irradiated with UV light by a UV irradiation device.

### Reference Signs List

1: air conditioner
2: heat exchanger
3: drain pan
4: drain pump
5: pipe
6: LED
7: suction port
8: discharge port
9: motor
10: control device
11: CPU
12: main memory
13: storage unit
14: external interface
15: communication interface
16: input unit
17: display unit
21: air conditioning control unit
22: sterilization control unit
23: irradiation control unit
24: pump control unit

## Claims

1. A sterilization system comprising:
an irradiation control unit that controls intermittent irradiation in which irradiation is performed for an irradiation time of which a length is equal to or shorter than 22% of a length of a reference time, the irradiation is stopped for a predetermined stoppage time after the irradiation time, and the irradiation is performed after the stoppage time, the reference time being a time taken for a predetermined sterilization rate to be reached in a case where drain water in a drain pan of an air conditioner is continuously irradiated with UV light by a UV irradiation device.

2. The sterilization system according to Claim 1,
wherein the irradiation control unit repeats performing irradiation for the irradiation time and stopping irradiation for the stoppage time.

3. The sterilization system according to Claim 2,
wherein the stoppage time is set to be equal to or longer than three times the irradiation time.

4. The sterilization system according to any one of Claims 1 to 3,
wherein the irradiation control unit performs irradiation with a predetermined amount of light, which is equal to or greater than 5000 mW.sec and equal to or smaller than 90000 mW.sec, per unit time in a case of the intermittent irradiation.

5. The sterilization system according to any one of Claims 1 to 4,
wherein the irradiation control unit performs the intermittent irradiation in a case where a predetermined standby time elapses after an end of the intermittent irradiation.

6. The sterilization system according to Claim 5,
wherein the irradiation control unit performs the intermittent irradiation in a case where cooling operation or dehumidification operation in the air conditioner is not performed for the standby time after the end of the intermittent irradiation.

7. The sterilization system according to any one of Claims 1 to 6, further comprising:
a pump control unit that operates, for a certain time, a drain pump that sucks the drain water in the drain pan.

8. The sterilization system according to any one of Claims 1 to 7,
wherein the irradiation control unit performs irradiation in a case where a sensor detects that the drain water is present in the drain pan.

9. An air conditioner comprising:
a drain pan;
a UV irradiation device; and
the sterilization system according to any one of Claims 1 to 8.

10. The air conditioner according to Claim 9,
wherein a useful life of the UV irradiation device is 5 or more and 10 or less in a case where a useful life of the air conditioner is 10.

11. The air conditioner according to Claim 10,
wherein the useful life is a rated life.

12. A sterilization method comprising:
performing irradiation for an irradiation time of which a length is equal to or shorter than 22% of a length of a reference time, the reference time being a time taken for a predetermined sterilization rate to be reached in a case where drain water in a drain pan of an air conditioner is continuously irradiated with UV light by a UV irradiation device;
stopping the irradiation for a predetermined stoppage time after the irradiation time; and
performing the irradiation after the stoppage time.

13. A sterilization program causing a computer to execute a process of performing irradiation for an irradiation time of which a length is equal to or shorter than 22% of a length of a reference time, stopping the irradiation for a predetermined stoppage time after the irradiation time, and performing the irradiation after the stoppage time, the reference time being a time taken for a predetermined sterilization rate to be reached in a case where drain water in a drain pan of an air conditioner is continuously irradiated with UV light by a UV irradiation device.
